(19) Europäisches Patentamt

European Patent Office

Office européen des brevets

(11) **EP 0 801 053 B1**

(12) **EUROPÄISCHE PATENTSCHRIFT**

(45) Veröffentlichungstag und Bekanntmachung des Hinweises auf die Patenterteilung:
**09.02.2000  Patentblatt 2000/06**

(51) Int Cl.[7]: **C07C 68/08**, C07C 68/00, C07C 69/96

(21) Anmeldenummer: **97105176.8**

(22) Anmeldetag: **27.03.1997**

(54) **Rückgewinnung von Katalysatorsystemen aus diarylcarbonathaltigen Reaktionslösungen durch Suspensionskristallisation**

Recovery of catalyst components from diarylcarbonate containing reaction solutions by suspension cristallization

Récupération de compositions catalytiques de solutions réactionnelles contenant du carbonate de diaryle par cristallisation de suspension

(84) Benannte Vertragsstaaten:
**BE DE ES FR GB IT NL**

(30) Priorität: **09.04.1996  DE 19613991**

(43) Veröffentlichungstag der Anmeldung:
**15.10.1997  Patentblatt 1997/42**

(73) Patentinhaber: **BAYER AG**
**51368 Leverkusen (DE)**

(72) Erfinder:
• **Buysch, Hans-Josef, Dr.**
**47809 Krefeld (DE)**
• **Hesse, Carsten Dr.**
**47800 Krefeld (DE)**
• **Rechner, Johann, Dr.**
**47906 Kempen (DE)**
• **Wirges, Hans-Peter, Dr.**
**47800 Krefeld (DE)**

(56) Entgegenhaltungen:
**EP-A- 0 687 666**      **US-A- 5 239 106**

## Beschreibung

Die vorliegende Erfindung betrifft ein Verfahren zur Rückgewinnung von Katalysatorsystemen aus diarylcarbonathaltigen Reaktionslösungen durch Suspensionskristallisation, wobei man eine das Katalysatorsystem enthaltende Mutterlauge und ein Kristallisat erhält. Die Mutterlauge kann in den Reaktor zur Herstellung des Diarylcarbonats zurückgeführt oder auf Wertstoffe aufgearbeitet werden. Das Kristallisat wird auf reines Diarylcarbonat und reine Hydroxyverbindung aufgearbeitet.

US 5 239 106 lehrt die Abtrennung von Diphenylcarbonat aus katalysatorhaltigen Reaktionslösungen durch Kristallisation des 1:1 Addukts mit Phenol, bestehend aus 30,5 Gew.-% Phenol und 69,5 Gew.-% Diphenylcarbonat, aus Reaktionsgemischen mit Hilfe der Suspensionskristallisation. Nachteilig bei diesem Verfahren ist die Beschränkung auf einen engen Konzentrationsbereich, um das 1:1-Addukt mit ausreichend hoher Ausbeute isolieren zu können, d. h. Diphenylcarbonatkonzentrationen von mindestens 50 Gew.-% bis 70 Gew.-%. Um die resultierenden Suspensionen filtrationstechnisch noch verarbeiten zu können, ist mindestens eine Zweistufigkeit des Verfahrens mit einen erheblichen apparativen Aufwand notwendig. Weiterhin läßt sich das Katalysatorsystem in diesem Verfahren nicht vollständig abtrennen, da die abfiltrierten Kristalle noch anhaftende Mutterlauge und Einschlüsse von Mutterlauge besitzen. Bei der anschließenden destillativen Aufarbeitung des 1:1-Addukts wirken sich diese nicht abgetrennten Katalysatorbestandteile durch die Katalyse von Nebenproduktbildung und der DPC-Zersetzung negativ aus. Die vorgeschlagene Waschung des Kristallisats mit einem Gemisch aus 9% Wasser und 91% Phenol reduziert die Ausbeute durch Lösen großer Teile des 1:1-Addukts. Weiterhin führt diese Behandlung zu einer Erhöhung des Wassergehalts der Addduktkristalle, was in den nachgeschalteten Destillationskolonnen, d.h. zur DPC-Isolierung und zur Wasserabtrennung aus der benutzten Waschlösung, zu DPC-Verlusten durch Hydrolyse führt. Weiterhin werden in US 5 239 106 wesentliche Verfahrensparameter, z.B. zur Art des Reaktors, zur Temperaturführung, Rührergeometrie, Rührleistung, usw., nicht offenbart.

Um auch Reaktionslösungen mit einem DPC-Gehalt von weniger als 50 Gew.-% mit diesem Verfahren verarbeiten zu können, ist eine destillative Anreicherung, mit den oben geschilderten Nachteilen einer Destillation in Anwesenheit von Katalysatorbestandteilen, unerläßlich. Zusätzlich führt eine thermische Belastung der Reaktionslösung zu einer Desaktivierung des Katalysatorsystems, was eine teure Frischeinspeisung der Katalysatorkomponenten in den Prozeß erfordert. Alle diese geschilderten Nachteile machen das Verfahren unflexibel, unattraktiv und stehen einer technischen Realisierung im Weg.

In EP-A 687 666 wird ein Verfahren zur Reinigung von Diphenylcarbonat durch fraktionierte Schmelzkristallisation von hochkonzentrierten Reaktionslösungen im Temperaturbereich von 45-85°C beschrieben. Die erzielbaren Diphenylcarbonatreinheiten liegen zwischen 97,5 und 99,5 %. Nachteilig an diesem Verfahren ist die Beschränkung auf Reaktionslösungen mit einem Diarylcarbonatgehalt von mehr als 70 Gew.-%. Reaktionslösungen mit Diarylcarbonatgehalten unter 70 Gew.-% lassen sich nach diesem Verfahren nicht verarbeiten und müßten daher beispielsweise durch Destillation auf die erforderlichen Gehalte aufkonzentriert werden. Bei dieser thermischen Belastung verursacht das Katalysatorsystem Nebenreaktionen und wird dabei selbst desaktiviert. Das Verfahren ist aus diesen Gründen für Reaktionslösungen mit Diphenylcarbonatgehalten unter 70 Gew.-% unwirtschaftlich und umständlich.

Es bestand daher die Aufgabe, eine schonende Methode zu finden, die es erlaubt, Katalysatorsysteme aus diarylcarbonathaltigen Reaktionslösungen mit unterschiedlichen Diarylcarbonatgehalten mit hoher Raum-Zeit-Ausbeute ohne Desaktivierung des Katalysatorsystems und unter wirtschaftlichen, technisch realisierbaren und reproduzierbaren Bedingungen abzutrennen und zurückzugewinnen.

Es wurde nun gefunden, daß die beschriebenen Nachteile überwunden werden können, wenn man die Reaktionslösung aus dem Reaktor entnimmt, in einer Suspensionskristallisation, bevorzugt durch Impfen der Reaktionslösung eine katalysatorhaltige Schmelze gewinnt, Reste des Katalysatorsystems aus dem Kristallisat mit einer wasserfreien Waschlösung, bevorzugt einem Gemisch aus Diarylcarbonat und aromatischer Hydroxyverbindung, wäscht, die so vom Katalysatorsystem und anderen Verunreinigungen befreiten Kristallisate, bestehend aus einem Gemisch aus Diarylcarbonat und aromatischer Hydroxyverbindung, verlustfrei durch Kristallisation oder Destillation in hochreines Diarylcarbonat aufarbeitet und die das Katalysatorsystem enthaltende Reaktionslösung in den Reaktor zurückführt. Die Waschlösung kann anschließend ohne weitere Behandlung der Reaktion als Feederganzung der aromatischen Hydroxyverbindung zugeführt werden. Überraschenderweise wurde im Falle des Diarylcarbonat/Phenol-Systems gefunden, daß sich die Zusammensetzung der Kristallisate in Abhängigkeit vom Diarylcarbonatgehalt der Reaktionslösung ändert und nur in einem sehr engen Konzentrationsbereich das 1:1-Addukt auftrat. Aus der Reaktionslösung wird nur soviel Diarylcarbonat durch Kristallisation abgetrennt, wie durch die Reaktion neu entsteht und zur Waschung der Kristallisate notwendig ist. Der Restgehalt an Diarylcarbonat wird gemeinsam mit den anderen Bestandteilen der Mutterlauge wieder dem Reaktor zugeführt. Eine thermische Schädigung des Katalysatorsystems findet nicht statt, was die Katalysatordesaktivierung auf ein Minimum reduziert. Ein besonderer Vorteil des erfindungsgemäßen Verfahrens besteht darin, daß Reaktionslösungen, deren Diarylcarbonatgehalt in einem weiten Bereich von 20 bis 70 % liegen, eingesetzt werden können. Außerdem können verschiedene Diarylcarbonate in den einsetzbaren Reaktionslösungen enthalten sein.

Die Erfindung betrifft demnach eine Methode zur Rückgewinnung von Katalysatorsystemen, die einen Platingruppenmetall-Katalysator, einen Cokatalysator, ein quaternäres Salz und eine Base enthalten, aus Reaktionslösungen zur Herstellung von Diarylcarbonaten der Formel

$$R\text{-}O\text{-}CO\text{-}O\text{-}R \qquad\qquad\qquad (I)$$

durch oxidative Carbonylierung der zugrundeliegenden aromatischen Hydroxyverbindungen der Formel

$$R\text{-}OH \qquad\qquad\qquad (II),$$

wobei in den Formeln

R  substituiertes oder nicht substituiertes $C_6$-$C_{15}$-Aryl, bevorzugt substituiertes oder nicht substituiertes Phenyl, besonders bevorzugt nicht substituiertes Phenyl bedeutet,
und die einen Diarylcarbonatgehalt von 20 bis 70 Gew.-%, bevorzugt 20 bis 50 Gew.-%, bezogen auf das Gesamtgewicht der Reaktionslösungen, aufweisen, die dadurch gekennzeichnet ist, daß man

    a) die Reaktionslösung aus dem Reaktor zur Herstellung des Diarylcarbonats in einen zur Suspensionskristallisation geeigneten Apparat überführt,

    b) im geeigneten Apparat durch Temperaturabsenkung die Suspensionskristallisation einleitet,

    c) das entstandene Kristallisat, das überwiegend aus Diarylcarbonat und der zugrundeliegenden aromatischen Hydroxyverbindung besteht, von der zurückbleibenden katalysatorhaltigen Mutterlauge abtrennt,

    d) das Kristallisat auf reines Diarylcarbonat und reine aromatische Hydroxyverbindung aufarbeitet und

    e) die katalysatorhaltige Mutterlauge in den Reaktor zur Herstellung von Diarylcarbonat zurückführt oder zur Gewinnung von Wertstoffen aufarbeitet.

R ist $C_6$-$C_{15}$-Aryl, wie Phenyl, Biphenylyl, Naphthyl, Anthryl oder HO-$C_6H_4$-C(CH$_3$)$_2$-$C_6H_4$- (Rest von Bisphenol A), bevorzugt Phenyl. Die aromatischen Kerne können jeweils ein- oder zweifach durch -CH$_3$, -C$_2$H$_5$, -Cl, -Br oder -F substituiert sein; besonders bevorzugtes R ist nicht substituiertes Phenyl.

Für das erfindungsgemäße Verfahren können Kristallisationstechnologien eingesetzt werden, wie sie beispielsweise in Chem.-Ing.-Techn. 57 (1985) 91 ff ausführlich beschrieben sind. Die häufig verwandten Rührkessel-Kristallisatoren, vergl. Chem.- Ing.-Techn. 57 (1985) S. 95, können nach Vorschriften und Empfehlungen von Ullmann's Encyclopedia of Industrial Chemistry, Vol B 2, Unit Operations I (1988), Chapter 25: Stirring, dimensioniert werden. Diese Verfahren werden diskontinuierlich oder kontinuierlich durchgeführt. Alle Apparate haben Wärmeaustauschflächen und einen Kühlmittelkreislauf; die unten angegebenen Temperaturen sind die des von den Wärmeaustauschflächen zurücklaufenden Kühlmittels.

Die folgenden Darstellungen sind beispielhaft auf Diphenylcarbonat (DPC) bezogen; es ist für den Fachmann jedoch einfach, die einzustellenden Bedingungen den physikalischen Daten anderer Diarylcarbonate anzupassen.

Beispielsweise kann die Suspensionskristallisation im diskontinuierlichen Rührwerkskristallisator mit einem Ankerrührer oder Kreuzbalkenrührer ohne Stromstörer und einer spezifischen Rührleistung P/V von 0,2 bis 0,5 W/l durchgeführt werden. Hierbei wird beispielsweise eine Kühlrate vom Kristallisationspunkt bis zur Kühlendtemperatur von 1 K/h eingestellt; es sind jedoch auch Kühlraten von 0,01 bis 20 K/h möglich. Die Anfangstemperatur für die Abkühlung ist von der Einwaagekonzentration, die Kühlendtemperatur ist von der gewünschten Suspensionskonzentration abhängig. Die bevorzugt durchgeführte Impfung erfolgt am Kristallisationspunkt mit DPC oder Addukt (0,02 bis 1 % bezogen auf eingesetztes DPC). Bei der Kühlendtemperatur wird bevorzugt eine Nachrührzeit von 1 bis 2 Stunden eingehalten. Die entstandene katalysatorhaltige Mutterlauge kann nach bekannten Verfahren, wie Abdekantieren, Abpressen, Zentrifugieren usw. vom Kristallisat getrennt werden. Das Kristallisat wird auf reines Diarylcarbonat und reine aromatische Hydroxyverbindung aufgearbeitet; dies kann beispielsweise durch Destillation, Lösungskristallisation, Extraktion oder andere bekannte Methoden geschehen. Die abgetrennte Mutterlauge wird gemeinsam mit beim Waschen erhaltenen weiteren Mutterlaugen als Katalysatorsystem in den Reaktor zur Herstellung des Diarylcarbonats zurückgeführt oder zur Gewinnung von Wertstoffen, etwa des Platingruppenmetalls, aufgearbeitet. Die Zurückführung ist die wichtigere Verwendung. In bevorzugter Weise wird das Kristallisat vor der Aufarbeitung auf reines Diarylcarbonat und reine aromatische Hydroxyverbindung mit einer wasserfreien Waschlösung gewaschen, beispielsweise durch Anschlämmen des Kristallisats in der Waschlösung. Diese Waschlösung ist bevorzugt eine systemeigene, nämlich ein Gemisch aus Diarylcarbonat und aromatischer Hydroxyverbindung oder die aromatische Hydroxyverbindung allein. Die Waschlösung wird mit der Mutterlauge der Kristallisation in bevorzugter Weise vereinigt und in die Herstellung des Diarylcarbonats zurückgeführt. In bevorzugter Weise ist die Waschlösung eine 10 bis 25 Gew.-%ige Lösung von Diarylcarbonat in der aromatischen Hydroxyverbindung. In weiter bevorzugter Weise wird die Anwendung der Waschlösung in einer Menge von 50 bis 250 Gew.-%, bezogen auf die Menge an Kristallisat, so durchgeführt, daß in mehreren Vorgängen Anteile der Waschlösung eingesetzt werden, beispielsweise so, daß das Kristallisat zunächst mit 25 bis 40 der Gesamtmenge Waschflüssigkeit angeschlämmt und dann filtriert wird und danach mit 60 bis 75 % der Gesamtmenge Waschflüssigkeit erneut angeschlämmt und dann filtriert wird.

Die nachfolgenden Beispiele sollen das Vorgehen verdeutlichen, ohne jedoch Einschränkungen zu begründen. Als Kernverunreinigung wird in den Beispielen Tetrabutylammoniumbromid (TBAB) betrachtet. TBAB stellt eine Leitsubstanz dar, die im Feed in der höchsten Konzentration auftritt und im Kristallisat als Reinigungsfaktor am leichtesten meßbar ist. Die Reaktionslösungen können nach bekannten Verfahren zur Herstellung von Diarylcarbonaten gewonnen werden, beispielsweise gemäß DE-A 19 605 167. In den folgenden Beispielen werden jedoch nur die relevanten Bestandteile DPC, Phenol und TBAB aufgeführt.

### Beispiele

Reaktionslösungen mit DPC, Phenol und TBAB, wie in den folgenden Tabellen angegeben (in Beispiel 1: 235,9 g DPC, 270,6 g Phenol, 5,2 g TBAB; Rest: Katalysator), wurden in einen Rührwerkskristallisator als Feed gegeben; durch den getakteten Einsatz mehrerer Kristaller kann eine quasi-kontinuierliche Fahrweise realisiert werden. Die Temperatur betrug am Anfang 53°C; die Temperatur beim Impfen und damit beim Kristallisationsbeginn ist jeweils angegeben. Nach einer Haltezeit wurde die Mutterlauge über einen Filter abgetrennt. Danach wurde das Kristallisat mit der angegebenen Waschflüssigkeit angeschlämmt und erneut abgetrennt; die dabei anfallende Waschlauge wurde mit der Mutterlauge vereinigt und zur Herstellung weiterer Reaktionslösungen eingesetzt (bei wiederholter Rückführung wird ein Anteil von einigen Prozent des Rückführstroms ausgeschleust und ersetzt). Das Kristallisat wurde destillativ aufgearbeitet. Die folgenden Tabellen zeigen detaillierte Angaben.

$$\text{*TBAB Abreicherungsfaktor} = \frac{\text{Masse TBAB Feed / Masse DPC Feed}}{\text{Masse TBAB Rein-Schmelze / Masse DPC Rein-Schmelze}}$$

### Beispiel 1: 46,0 % DPC

| Kristallisationsbedingungen | | |
|---|---|---|
| Kristallisationstemperatur | [°C] | 46 |
| Impftemperatur | [°C] | 46 |
| Kühlendtemperatur | [°C] | 43 |
| Kühlrate | [K/h] | 1 |
| Kühlzeit | [h] | 3 |
| Haltezeit | [h] | 2 |

| | Masse [g] | DPC [g] | Phenol [g] | TBAB [g] | Abreicherungs-Faktor TBAB |
|---|---|---|---|---|---|
| Feed | 512,9 | 235,9 | 270,6 | 5,2 | |
| Waschflüssigkeit | 300,0 | 60,0 | 240,0 | - | |
| Mutterlauge | 248,9 | 88,0 | 157,0 | 3,9 | |
| Waschlauge | 414,0 | 115,9 | 295,6 | 1,3 | |
| Kristallisat | 150,0 | 92,0 | 58,0 | 0,03 | 70 |

| Raum-Zeit-Ausbeute | [kg/m3∗h] | 36,1 |
|---|---|---|
| DPC-Ausbeute | [%] | 39,0 |

**Beispiel 2: 44,3 % DPC**

| Kristallisationsbedingungen | | |
|---|---|---|
| Kristallisationstemperatur | [°C] | 45 |
| Impftemperatur | [°C] | 45 |
| Kühlendtemperatur | [°C] | 43 |
| Kühlrate | [K/h] | 1 |
| Kühlzeit | [h] | 2 |
| Haltezeit | [h] | 2 |
| | | |

| | Masse [g] | DPC [g] | Phenol [g] | TBAB [g] | Abreicherungs-Faktor TBAB |
|---|---|---|---|---|---|
| Feed | 570,3 | 252,4 | 310,2 | 6,3 | |
| Waschflüssigkeit | 200,0 | 40,0 | 160,0 | - | |
| Mutterlauge | 321,1 | 113,2 | 203,0 | 4,9 | |
| Waschlauge | 278,2 | 77,2 | 198,2 | 1,3 | |
| Kristallisat | 171,0 | 102,0 | 69,0 | 0,05 | 47 |

| Raum-Zeit-Ausbeute | [kg/m3∗h] | 44,7 |
|---|---|---|
| DPC-Ausbeute | [%] | 40,5 |

**Beispiel 3: 46,9 % DPC, <u>Filtration ohne Wäsche</u>**

| Kristallisationsbedingungen | | |
|---|---|---|
| Kristallisationstemperatur | [°C] | 46 |
| Impftemperatur | [°C] | 46 |
| Kühlendtemperatur | [°C] | 41 |
| Kühlrate | [K/h] | 1 |
| Kühlzeit | [h] | 5 |
| Haltezeit | [h] | 2 |

| | Masse [g] | DPC [g] | Phenol [g] | TBAB [g] | Abreicherungs-Faktor TBAB |
|---|---|---|---|---|---|
| Feed | 520,2 | 243,8 | 269,5 | 5,5 | |
| Mutterlauge | 317,5 | 121,5 | 189,3 | 5,26 | |
| Kristallisat | 202,7 | 122,3 | 80,2 | 0,24 | 11,3 |

EP 0 801 053 B1

| Raum-Zeit-Ausbeute | [kg/m3∗h] | 34,3 |
|---|---|---|
| DPC-Ausbeute | [%] | 50,1 |

**Patentansprüche**

1. Methode zur Rückgewinnung von Katalysatorsystemen, die einen Platingruppenmetall-Katalysator, einen Cokatalysator, ein quaternäres Salz und eine Base enthalten, aus Reaktionslösungen zur Herstellung von Diarylcarbonaten der Formel

$$R\text{-}O\text{-}CO\text{-}O\text{-}R \qquad (I)$$

durch oxidative Carbonylierung der zugrundeliegenden aromatischen Hydroxyverbindungen der Formel

$$R\text{-}OH \qquad (II),$$

wobei in den Formeln

R substituiertes oder nicht substituiertes $C_6$-$C_{15}$-Aryl, bedeutet,

und die einen Diarylcarbonatgehalt von 20 bis 70 Gew.-%, bezogen auf das Gesamtgewicht der Reaktionslösungen, aufweisen, dadurch gekennzeichnet, daß man

a) die Reaktionslösung aus dem Reaktor zur Herstellung des Diarylcarbonats in einen zur Suspensionskristallisation geeigneten Apparat überführt,

b) im geeigneten Apparat durch Temperaturabsenkung die Suspensionskristallisation einleitet,

c) das entstandene Kristallisat, das überwiegend aus Diarylcarbonat und der zugrundeliegenden aromatischen Hydroxyverbindung besteht, von der zurückbleibenden katalysatorhaltigen Mutterlauge abtrennt,

d) das Kristallisat auf reines Diarylcarbonat und reine aromatische Hydroxyverbindung aufarbeitet und

e) die katalysatorhaltige Mutterlauge in den Reaktor zur Herstellung von Diarylcarbonat zurückführt oder zur Gewinnung von Wertstoffen aufarbeitet.

2. Verfahren nach Anspruch 1, dadurch gekennzeichnet, daß die Temperatur der Reaktionslösung mit einer Kühlrate von 20 bis 0,01 K/h, abgesenkt wird.

3. Verfahren nach Anspruch 1, dadurch gekennzeichnet, daß im Schritt b) das Einleiten der Suspensionskristallisation außer durch Temperaturabsenkung zusätzlich durch Impfen mit festem Diarylcarbonat, fester aromatischer Hydroxyverbindung oder einem Gemisch beider als Impfgut vorgenommen wird.

4. Verfahren nach Anspruch 3, dadurch gekennzeichnet, daß 0,02 bis 1 Gew.-% Impfgut, bezogen auf in der Reaktionslösung vorliegendes Diarylcarbonat, eingesetzt werden.

5. Verfahren nach Anspruch 3, dadurch gekennzeichnet, daß zum Impfen Kristallisat aus einem vorangegangenen Reaktionslauf eingesetzt wird.

6. Verfahren nach Anspruch 1, dadurch gekennzeichnet, daß man im Schritt c) das Kristallisat nach der Abrennung der Mutterlauge mit einer wasserfreien Waschlösung wäscht.

7. Verfahren nach Anspruch 6, dadurch gekennzeichnet, daß man als Waschlösung ein Gemisch aus Diarylcarbonat und aromatischer Hydroxyverbindung oder die aromatische Hydroxyverbindung allein einsetzt und die Waschlösung in die Herstellung von Diarylcarbonat zurückführt.

8. Verfahren nach Anspruch 3, dadurch gekennzeichnet, daß die Waschlösung eine 10 bis 25 Gew.-%ige Lösung von Diarylcarbonat in aromatischer Hydroxyverbindung darstellt.

9. Verfahren nach Anspruch 8, dadurch gekennzeichnet, daß die Waschlösung in einer Menge von 50 bis 250 Gew.-%, bezogen auf die Menge Kristallisat eingesetzt wird, wobei das Kristallisat zunächst mit 25 bis 40 der Gesamtmenge Waschflüssigkeit angeschlämmt und dann filtriert wird und danach mit 60 bis 75 % der Gesamtmenge Waschflüssigkeit erneut angeschlämmt und dann filtriert wird.

**Claims**

1. Method for recovering catalyst systems containing a platinum group metal catalyst, a co-catalyst, a quaternary salt and a base from reaction solutions for the production of diaryl carbonates of the formula

$$R\text{-}O\text{-}CO\text{-}O\text{-}R \qquad\qquad (I)$$

by oxidative carbonylation of the underlying aromatic hydroxy compounds of the formula

$$R\text{-}OH \qquad\qquad (II),$$

wherein in the formulae

R   means substituted or unsubstituted $C_6$-$C_{15}$ aryl,

and which have a diaryl carbonate content of 20 to 70 wt.%, relative to the total weight of the reaction solutions, characterised in that

  a) the reaction solution is transferred from the reactor for the production of the diaryl carbonate into an apparatus suitable for suspension crystallisation,

  b) suspension crystallisation is initiated in the suitable apparatus by reducing the temperature,

  c) the resultant crystallisate, which primarily consists of diaryl carbonate and the underlying aromatic hydroxy compound, is separated from the remaining mother liquor containing catalyst,

  d) the crystallisate is worked up to yield pure diaryl carbonate and pure aromatic hydroxy compound and

  e) the mother liquor containing catalyst is returned to the reactor for the production of the diaryl carbonate or is worked up in order to isolate valuable materials.

2. Process according to claim 1, characterised in that the temperature of the reaction solution is lowered at a cooling rate of 20 to 0.01 K/h.

3. Process according to claim 1, characterised in that in stage b) suspension crystallisation is initiated, apart from by reducing the temperature, additionally by seeding with solid diaryl carbonate, solid aromatic hydroxy compound or a mixture of the two as seeding material.

4. Process according to claim 3, characterised in that 0.02 to 1 wt.% of seeding material, relative to the diaryl carbonate present in the reaction solution, is used.

5. Process according to claim 3, characterised in that crystallisate from a preceding reaction run is used for seeding.

6. Process according to claim 1, characterised in that in stage c) the crystallisate is washed with an anhydrous washing solution once the mother liquor has been separated.

7. Process according to claim 6, characterised in that the washing solution used is a mixture of diaryl carbonate and aromatic hydroxy compound or the aromatic hydroxy compound alone and the washing solution is returned to the production of the diaryl carbonate.

8. Process according to claim 3, characterised in that the washing solution is a 10 to 25 wt.% solution of diaryl carbonate in aromatic hydroxy compound.

9. Process according to claim 8, characterised in that the washing solution is used in a quantity of 50 to 250 wt.%, relative to the quantity of crystallisate, wherein the crystallisate is first suspended with 25 to 40% of the total quantity of washing fluid and is then filtered and is subsequently resuspended with 60 to 75% of the total quantity of washing fluid and then filtered.

## Revendications

1. Procédé pour récupérer des systèmes catalyseurs contenant un catalyseur à base d'un métal du groupe du platine, un catalyseur auxiliaire, un sel quaternaire et une base, à partir de solutions de réaction de la préparation de carbonates de diaryle de formule

$$R\text{-}O\text{-}CO\text{-}O\text{-}R \qquad\qquad (I)$$

par carbonylation oxydative des composés aromatiques hydroxylés correspondants de formule

$$R\text{-}OH \qquad\qquad (II),$$

R représentant dans les deux formules un groupe aryle en $C_6$-$C_{15}$ substitué ou non,

et qui ont une teneur en carbonate de diaryle de 20 à 70 % de leur poids, caractérisé en ce que

a) on transfère la solution de réaction, du réacteur de préparation du carbonate de diaryle, dans un appareil approprié à la cristallisation en suspension,
b) on déclenche la cristallisation en suspension dans cet appareil en abaissant la température,
c) on sépare les cristaux formés, consistant principalement en le carbonate de diaryle et le composé aromatique hydroxylé correspondant, des liqueurs-mères résiduelles contenant le catalyseur,
d) on isole à partir des cristaux le carbonate de diaryle pur et le composé aromatique hydroxylé pur et
e) on recycle les liqueurs-mères contenant le catalyseur au réacteur de préparation du carbonate de diaryle, ou on les traite pour isolement de substances de valeur.

2. Procédé selon la revendication 1, caractérisé en ce que la température de la solution de réaction est abaissée à une vitesse de 20 à 0,01 K/h.

3. Procédé selon la revendication 1, caractérisé en ce que, au stade opératoire b), outre l'abaissement de température, on déclenche la cristallisation en suspension par ensemencement à l'aide du carbonate de diaryle solide, du composé aromatique hydroxylé solide ou d'un mélange des deux substances, servant de germes de cristallisation.

4. Procédé selon la revendication 3, caractérisé en ce que l'on utilise 0,02 à 1 % en poids de germes de cristallisation par rapport au carbonate de diaryle présent dans la solution de réaction.

5. Procédé selon revendication 3, caractérisé en ce que l'on ensemence à l'aide de cristaux obtenus dans une opération précédente.

6. Procédé selon la revendication 1, caractérisé en ce que, au stade opératoire c), et après séparation des liqueurs-mères, on lave les cristaux à l'aide d'une solution de lavage anhydre.

7. Procédé selon la revendication 6, caractérisé en ce que l'on utilise en tant que solution de lavage un mélange du carbonate de diaryle et du composé aromatique hydroxylé ou le composé aromatique hydroxylé seul et on recycle la solution de lavage à la préparation du carbonate de diaryle.

8. Procédé selon la revendication 3, caractérisé en ce que la solution de lavage consiste en une solution de carbonate de diaryle à la concentration de 10 à 25 % en poids dans le composé aromatique hydroxylé.

9. Procédé selon la revendication 8, caractérisé en ce que la solution de lavage est utilisée en quantité de 20 à 250 % du poids des cristaux, en ce que ceux-ci sont d'abord dispersés dans 25 à 40 % de la quantité totale de liquide de lavage puis filtrés et à nouveau redispersés dans 60 à 75 % de la quantité totale de liquide de lavage et à nouveau filtrés.